# EUROPEAN PATENT APPLICATION

(11) **EP 0 669 124 A1**
(43) Date of publication of application: **30.08.1995**
(21) Application number: 95300967.7
(22) Date of filing: 15.02.1995
(51) Int. Cl.: A61K 7/42

(54) **Ultraviolet radiation absorbing composition**

(30) Priority: 28.02.1994 US 203178
(71) Applicant: ROHM AND HAAS COMPANY, Philadelphia, Pennsylvania 19106-2399 (US)
(72) Inventor: Jones, Charles Elwood, Yardley, Pennsylvania 19067 (US); Fasano, David Michael, Maple Glenn, Pennsylvania 19002 (US); Aviles, Rafael Gonzalez, Harleysville, Pennsylvania 19438 (US); Vogel, Martin, Jenkintown, Pennsylvania 19046 (US)
(74) Representative: Angell, David Whilton

(57) **Abstract**

A composition is disclosed containing at least one UV radiation absorbing agent and about 0.1% to about 50%, based on total weight of nonvolatiles, of latex particles having a particle size of from about 50 to about 500 nm. The latex particles are preferably voided, having a void fraction of from 0.1 to 50%.

## Description

This invention relates to compositions containing ultraviolet radiation absorbing materials and, more particularly, to compositions containing ultraviolet absorbing agents and latex particles.

Six percent of the solar energy reaching the earth's surface is ultraviolet (UV) radiation having a wavelength of 290-400 nanometers (nm). This radiation has two components:
(1) 5.5% UVA having a wavelength of 320-400 nm and
(2) 0.5% UVB having a wavelength of 290-320 nm.

While the UV portion of the solar energy is relatively small, it induces nearly 99% of all the side effects of sunlight. UVB radiation, for example, is responsible for producing sunburn, aging and cancer of the skin. UVA radiation, for example, causes direct tanning and erythema (abnormal redness) and contributes to aging of the skin.

Protection from these harmful effects of UV radiation exposure is available in the form of both topically applied formulations containing at least one physical blocker and/or at least one chemical absorber. Physical blockers include active ingredients such as red petrolatum, titanium dioxide and zinc oxide. Chemical absorbers include active ingredients, such as para-aminobenzoic acid (more commonly known as PABA), which are generally transparent when applied and act by absorbing UV radiation, offering selective protection against certain UV wave bands, depending upon the absorption spectrum of the particular active ingredient incorporated into the formulation.

The effectiveness of a sunscreen formulation in protecting the skin is generally defined in terms of a Sun Protection Factor (SPF), which is defined as the ratio of the amount of energy required to produce a minimal erythema on sunscreen protected skin to the amount of energy required to produce the same level of erythema on unprotected skin.

A number of the active ingredients and physical blockers typically used in sunscreen formulations, hereinafter referred to as "UV radiation absorbing agents", have poor toxicological effects. Therefore, it is generally desirable to reduce the level of active ingredient present in a sunscreen formulation without reducing the level of protection.

We have discovered that the addition of from about 0.1% to about 50%, based on total weight of nonvolatiles, of latex particles having a particle size of from about 50 to about 500 nanometers (nm) to compositions containing at least one ultraviolet radiation absorbing agent improves the UV radiation absorption of the composition.

Accordingly the present invention provides a composition comprising at least one UV radiation absorbing agent, which further comprises from 0.1% to 50%, based on total weight of nonvolatiles, of latex particles having a particle size of from 50 to 500 nm. A further aspect of the invention provides a method of improving the UV radiation absorption of a composition containing a UV radiation absorbing agent, comprising incorporating into said composition from 0.1% to 50%, based on total weight of nonvolatiles, of latex particles as defined above. The invention also comprises the use of the above latex particles to improve the UV radiation absorption of a composition containing a UV radiation absorbing agent.

### In the drawings:

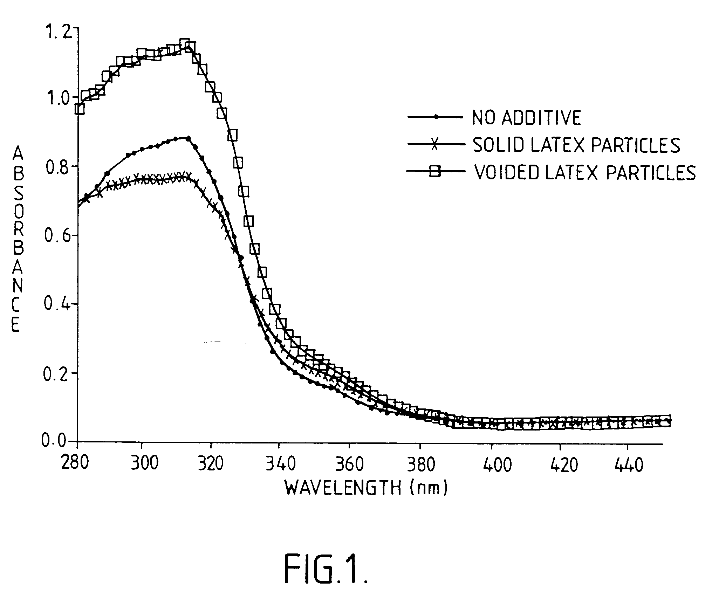

**FIG. 1** is a UV radiation absorbance spectrum at wavelengths of 280-440 nm for compositions containing no additives, solid latex particles and voided latex particles at a coating levels of 5 microliters per square inch (µl/in²), or 0.8 µl/cm². The active ingredients in the compositions are 2-ethylhexyl para-methoxycinnamate and menthyl anthranilate. The additives are incorporated at a level of 5% by weight, based on total weight nonvolatiles.

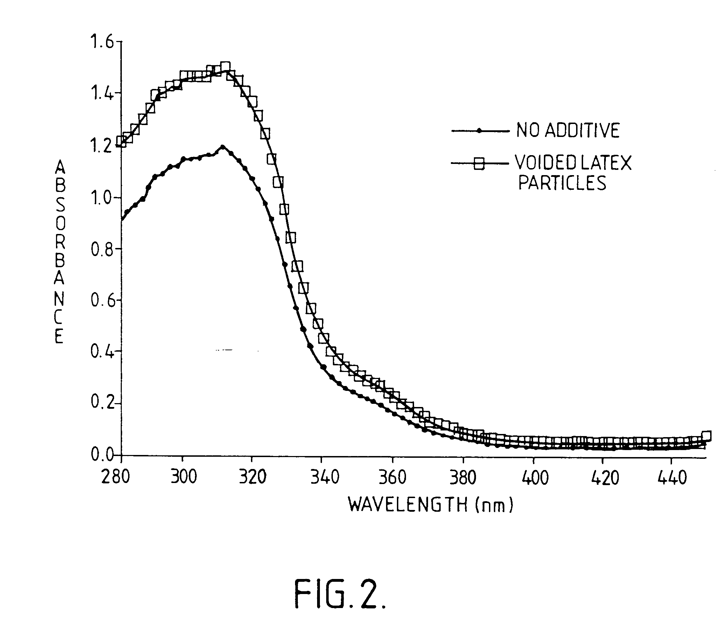

**FIG. 2** is a UV radiation absorbance spectrum at wavelengths of 280-440 nm for compositions containing no additives and voided latex particles at a coating levels of 10 µl/in² (1.5 µl/cm²). The active ingredients in the compositions are 2-ethylhexyl para-methoxycinnamate and menthyl anthranilate. The additives are incorporated at a level of 5% by weight, based on total weight nonvolatiles.

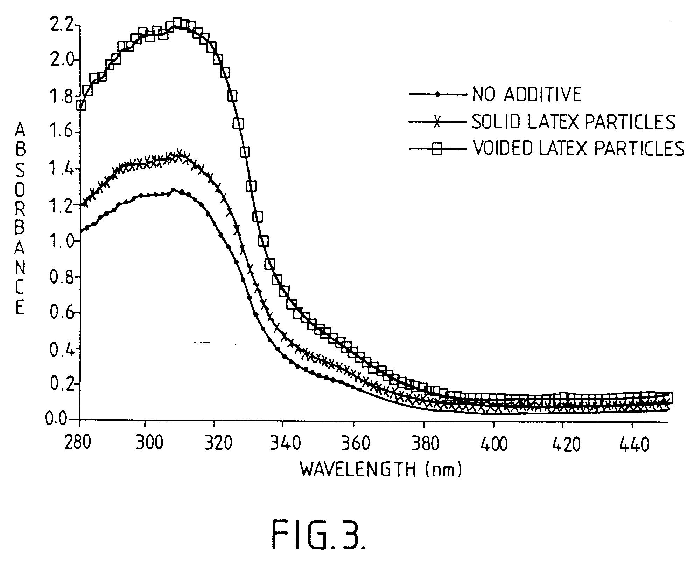

**FIG. 3** is a UV radiation absorbance spectrum from a wavelength of 280-440 nm for compositions containing no additives, solid latex particles and voided latex particles at a coating levels of 20 µl/in² (3.1 µl/cm²). The active ingredients in the compositions are 2-ethylhexyl para-methoxycinnamate and menthyl anthranilate. The additives are incorporated at a level of 5% by weight, based on total weight nonvolatiles.

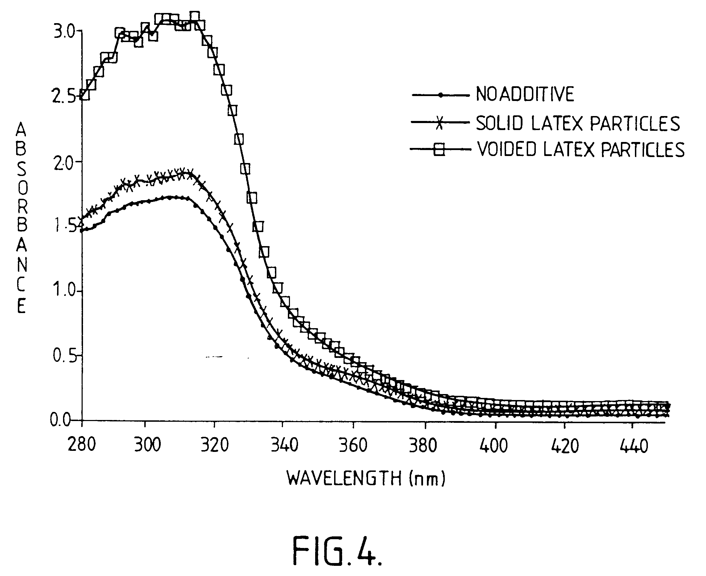

**FIG. 4** is a UV radiation absorbance spectrum from a wavelength of 280-440 nm for compositions containing no additives, solid latex particles and voided latex particles at a coating levels of 40 µl/in² (6.2 µl/cm²). The active ingredients in the compositions are 2-ethylhexyl para-methoxycinnamate and menthyl anthranilate. The additives are incorporated at a level of 5% by weight, based on total weight nonvolatiles.

The composition of this invention contains at least two components:
(1) at least one UV radiation absorbing agent and
(2) latex particles.

Compositions containing at least one UV radiation absorbing agent and solid latex particles exhibit increased UV radiation absorbance compared with compositions containing only a UV radiation absorbing agent. Compositions containing at least one UV radiation absorbing agent and voided latex particles exhibit still better UV radiation absorbance. As used herein, the term "UV radiation" includes both UVA and UVB radiation.

The invention improves the UV radiation absorbance of compositions containing UV radiation absorbing agents. It involves incorporating from about 0.1% to about 50%, based on total weight nonvolatiles, of latex particles having a particle size of from about 50 to about 500 nanometres (nm) to compositions containing at least one ultraviolet radiation absorbing agent. Preferably, the latex particles are voided and have a void fraction of from about 0.1% to about 50%, to compositions containing at least one ultraviolet radiation absorbing agent.

The composition of this invention may be used in either colourless or pigmented formulations. The composition is particularly useful if a colourless formulation is desired, such as for a sunscreen formulation, because the addition of the voided latex particles of the invention having a void diameter of less than about 150 nm does not contribute to opacity.

The UV absorbing agents useful in the composition and method of this invention are conventional materials. Suitable UV radiation absorbing agents include oxybenzone, dioxybenzone, sulisobenzone, menthyl anthranilate, para-aminobenzoic acid, amyl dimethyl-para-aminobenzoic acid, octyl dimethyl para-aminobenzoate, ethyl 4-bis (hydroxypropyl) para-aminobenzoate, polyethylene glycol (PEG-25) para-aminobenzoate, ethyl 4-bis (hydroxypropyl) aminobenzoate, diethanolamine para-methyoxycinnamate, 2-ethoxyethyl para-methoxycinnamate, ethylhexyl para-methoxycinnamate, octyl paramethoxycinnamate, isoamyl para-methoxycinnamate, 2-ethylhexyl 2-cyano-3,3-diphenyl-acrylate, 2-ethylhexyl salicylate, homomenthyl salicylate, glyceryl aminobenzoate, triethanolamine salicylate, digalloyl trioleate, lawsone with dihydroxyacetone, 2-phenylbenzimidazole-5-sulfonic acid, benzylidine camphor, avobenzone, titanium dioxide and zinc oxide and the like.

The latex particles useful in this invention have a particle size of from about 50 to about 500 nanometres (nm), preferably from about 100 to about 250nm, as measured by the Brookhaven BI-90 photon correlation spectrometer. Preferably, the latex particles contain a void with a void fraction of from about 0.1% to about 50%, preferably from about 5% to about 20%, as determined by comparing the volume occupied by the latex particles after they have been compacted from a dilute dispersion in a centrifuge to the volume of non-voided particles of the same composition.

The latex particles may be prepared by conventional polymerization techniques such as, sequential emulsion polymerization. The voided latex particles are formed from a multistaged particle having a core polymer and a shell polymer. They may be prepared by conventional polymerization techniques such as, sequential emulsion polymerization, including those processes disclosed in U.S. 4,497,836; 4,468,498; 4,469,825; 4,594,363; 4,677,003 4,920,160; 4,970,241, whose disclosures are incorporated herein by reference, and EP-A-267726.

The monomers used in the emulsion polymerization of the shell polymer of the voided latex particles may be one or more monoethylenically unsaturated monomers containing at least one carboxylic acid group and one or more non-ionic ethylenically unsaturated monomer.

The monomers used in the emulsion polymerization of the core polymer of the voided latex particles may be of one or more monoethylenically unsaturated monomers containing at least one carboxylic acid group. The core polymer may be obtained by the emulsion homopolymerization of such a monoethylenically unsaturated monomer containing at least one carboxylic acid group or by copolymerization of two or more monoethylenically unsaturated monomers containing at least one carboxylic acid group. Preferably, the monoethylenically unsaturated monomer containing at least one carboxylic acid group is copolymerized with one or more non-ionic (that is, having no ionizable group) ethylenically unsaturated monomers.

The core polymer may also contain from about 0.1% to about 20% by weight, based on the weight of the total monomer weight of the core, preferably about 0.1% to about 3% by weight of polyethylenically unsaturated monomer, such as ethylene glycol di(meth)acrylate, allyl (meth)acrylate, 1,3-butanediol di(meth)acrylate, diethylene glycol di(meth)acrylate, trimethylolpropane trimethacrylate, or divinylbenzene. Alternatively, the core polymer may contain from about 0.1% to about 60% by weight, based on the weight of the total monomer weight of the core, of butadiene.

Suitable monoethylenically unsaturated monomers containing at least one carboxylic acid group include acrylic acid and methacrylic acid, acryloxypropionic acid, (meth)acryloxypropionic acid, itaconic acid, aconitic acid, maleic acid or anhydride, fumaric acid, crotonic acid, monomethyl maleate, monomethyl fumarate, and monomethyl itaconate. Acrylic acid and methacrylic acid are preferred.

Suitable non-ionic ethylenically unsaturated monomers include styrene, vinyltoluene, ethylene, vinyl acetate, vinyl chloride, vinylidene chloride, acrylonitrile, (meth)acrylamide, (C₁-C₂₀) alkyl or (C₃-C₂₀) alkenyl esters of (meth)acrylic acid, such as methyl (meth)acrylate, ethyl (meth)acrylate, butyl (meth)acrylate, 2-ethylhexyl (meth)acrylate, benzyl (meth)acrylate, lauryl (meth)acrylate, oleyl (meth)acrylate, palmityl (meth)acrylate and stearyl (meth)acrylate. As used herein, the term "(meth)acrylic" is intended to serve as a generic expression embracing both acrylic and methacrylic.

The latex particles are incorporated into the composition at a level of from about 0.1% to about 50%, based on total weight of nonvolatiles, and preferably from about 1% to about 10%, based on total weight of nonvolatiles.

The compositions of this invention are useful in improving the ultraviolet radiation absorption by compositions containing UV absorbing materials used on human skin and hair, such as, for example personal care products, including, but not limited to, cosmetics, sunscreens and hair care products. In addition, they are useful in improving the UV absorption and protection for coatings on plant life, plastics, wood, for example in the form of a clear varnish, and the like.

The composition of this invention may include other conventional ingredients. For example, if the composition is used as a sunscreen, it may additionally include water, film forming materials, emulsifiers, water, emollients, waterproofing agents, oils, stabilizers, thickeners, preservatives, perfume, colorants, insecticides, humectants and the like. If the composition is used as a cosmetic, it may additionally include, water, film forming materials, emulsifiers, softeners, emollients, oils, stabilizers, thickeners, preservatives, perfume, colorants, pigments and the like.

The invention enables formulators either to increase the UV radiation absorbance of a given formulation or to reduce the level of the UV radiation absorbing agent present in the formulation while maintaining a given UV radiation absorbance.

The compositions of the invention may be applied to the skin at coating volumes, for example, of about 2 microlitres per square centimetre.

The following examples illustrate the operation of the invention using conventional UV radiation absorbing agents. These examples are intended merely to illustrate the invention and are not intended nor should they be interpreted as limiting the scope of the invention.
**Note:** The following abbreviations are used in the Examples:
- MMA: methyl methacrylate
- BMA: butyl methacrylate
- MAA: methacrylic acid
- Sty: styrene
- ALMA: allyl methacrylate

### EXAMPLE 1. PREPARATION OF VOIDED LATEX PARTICLES

The voided latex particles useful in the invention were prepared by the method described in US 4,427,836. They have a composition of:
1 part (60MMA/40MAA) [39 nm core] //
16 parts (10BMA/86MMA/4MAA) [shell I] //
12 parts (99.5Sty/0.5 ALMA) [shell II] //
9 parts (100Sty) [shell III].
Excess ammonia (1.3 equivalents based on the total equivalents of methacrylic acid in the particles) was added to the hot (80-85°C) dispersion between the polymerization of shell II and shell III to convert the acid-rich core to the ammonium salt causing the core to swell with water. The particles had a final particle size of 160 nm, as measured by the Brookhaven BI-90 photon correlation spectrometer, and a void fraction of 10%, as measured by the centrifuge method described above.

### EXAMPLE 2. PREPARATION OF COMPOSITIONS

Three different compositions were prepared for UV radiation absorbance measurements:
Comparative Composition A containing no additive,
Composition 1 containing as additive solid polystyrene particles having a particle size of about 179 nm, and
Composition 2 containing as additive the voided latex particles prepared in Example 1.
The compositions were prepared by adding the additive at a level of 5% solids, based on total weight solids, to Hawaiian Tropic Dark Tanning Lotion with an SPF of 4, manufactured by Tanning Research Laboratories. The UV absorbing materials in the Hawaiian Tropic Dark Tanning Lotion were 2-ethylhexyl para-methoxycinnamate and menthyl anthranilate.

### EXAMPLE 3. PERFORMANCE TESTING

The compositions prepared according to the procedure of Example 2 were coated at a level of 5, 10, 20 and 40 µl/in² (0.8, 1.5, 3.1 and 6.2 µl/cm²) on Transpore® tape from Minnesota Mining and Manufacturing Company to simulate different levels of sunscreen on human skin. The UV radiation absorbance spectra from a wavelength of 280-440 nm for each sample were measured using an Optronics Laboratories 752 Spectroradiometer. The spectra at coating levels of 5, 10, 20 and 40 µl/in² are shown in Figs. 1, 2, 3 and 4 respectively.

Composition 2 containing both UV radiation absorbing agent and voided latex particles exhibited increased UV radiation absorbance at each coating level over the wavelengths tested (280-440 nm) compared with the composition containing only a UV radiation absorbing agent (Comparative Composition A) and also compared with the composition containing a UV radiation absorbing agent and solid latex particles of the similar particle size to the voided latex particles (Composition 1). Composition 1 itself exhibited increased UV radiation absorbance at coating levels of 20 and 40 µl/in² over the wavelengths tested (280-440 nm) compared with Comparative Composition A.

## Claims

1. Composition comprising at least one UV radiation absorbing agent, which further comprises from 0.1% to 50%, based on total weight of nonvolatiles, of latex particles having a particle size of from 50 to 500 nm.

2. Composition according to claim 1 comprising from 1 to 10 weight % of latex particles.

3. Composition according to claim 1 or 2 wherein the latex particles contain a void, the void fraction being from 0.1% to 50%, preferably from 5% to 20%.

4. Composition according to any preceding claim wherein the latex particles have a particle size of from 100 to 250 nm.

5. Composition according to any claims 3 or 4 wherein the voided latex particles comprise a core polymer and a shell polymer, the shell polymer being formed from one or more of acrylic acid, methacrylic acid, acryloxypropionic acid, (meth)acryloxypropionic acid, itaconic acid, aconitic acid, maleic acid or anhydride, fumaric acid, crotonic acid, monomethyl maleate, monomethyl fumarate, or monomethyl itaconate, preferably acrylic or methacrylic acid, and one or more of styrene, vinyltoluene, ethylene, vinyl acetate, vinyl chloride, vinylidene chloride, acrylonitrile, (meth)acrylamide, (C₁-C₂₀) alkyl or (C₃-C₂₀) alkenyl esters of(meth)acrylic acid, such as methyl (meth)acrylate, ethyl (meth)acrylate, butyl (meth)acrylate, 2-ethylhexyl (meth)acrylate, benzyl (meth)acrylate, lauryl (meth)acrylate, oleyl (meth)acrylate, palmityl (meth)acrylate or stearyl (meth)acrylate..

6. Composition according to any one of claims 3 to 5 wherein the voided latex particles comprise a core polymer and a shell polymer, the core polymer being formed from one or more of acrylic acid, methacrylic acid, acryloxypropionic acid, (meth)acryloxypropionic acid, itaconic acid, aconitic acid, maleic acid or anhydride, fumaric acid, crotonic acid, monomethyl maleate, monomethyl fumarate, or monomethyl itaconate; preferably acrylic or methacrylic acid.

7. Composition according to any claim 5 or 6 wherein the core polymer additionally comprises either:
from 0.1% to 20% by weight, based on the weight of the total monomer weight of the core, preferably 0.1% to 3% by weight, of polyethylenically unsaturated monomer, preferably ethylene glycol di(meth)acrylate, allyl (meth)acrylate, 1,3-butanediol di(meth)acrylate, diethylene glycol di(meth)acrylate, trimethylolpropane trimethacrylate, or divinylbenzene; or from 0.1% to 60% by weight, based on the weight of the total monomer weight of the core, of butadiene.

8. Composition according to any preceding claim wherein the UV radiation absorbing agent comprises oxybenzone, dioxybenzone, sulisobenzone, menthyl anthranilate, para-aminobenzoic acid, amyl dimethyl-para-aminobenzoic acid, octyl dimethyl para-aminobenzoate, ethyl 4-bis (hydroxypropyl) para-aminobenzoate, polyethylene glycol (PEG-25) para-aminobenzoate, ethyl 4-bis (hydroxypropyl) aminobenzoate, diethanolamine para-methyoxycinnamate, 2-ethoxyethyl para-methoxycinnamate, ethylhexyl pa-methoxycinnamate, octyl paramethoxycinnamate, isoamyl para-methoxycinnamate, 2-ethylhexyl 2-cyano-3,3-diphenyl-acrylate, 2-ethylhexyl salicylate, homomenthyl salicylate, glyceryl aminobenzoate, triethanolamine salicylate, digalloyl trioleate, lawsone with dihydroxyacetone, 2-phenylbenzimidazole-5-sulfonic acid, benzylidine camphor, avobenzone, titanium dioxide or zinc oxide.

9. A method of improving the UV radiation absorption of a composition containing a UV radiation absorbing agent, comprising incorporating into said composition from 0.1% to 50%, based on total weight of nonvolatiles, of latex particles as defined in any of claims 1 to 7.

10. The use of latex particles as defined in any of claims 1 to 7 to improve the UV radiation absorption of a composition containing a UV radiation absorbing agent.
